# EUROPEAN PATENT APPLICATION

(11) **EP 4 759 237 A1**
(43) Date of publication of application: **17.06.2026**
(21) Application number: 24220134.1
(22) Date of filing: 16.12.2024
(51) Int. Cl.: A61B 6/00

(54) **SCATTER CORRECTION FOR X-RAY IMAGING WITH CAMERA INFORMATION**

(71) Applicant: Koninklijke Philips N.V., 5656 AE Eindhoven (NL)
(72) Inventor: GRASS, Michael, Eindhoven (NL); VON BERG, Jens, Eindhoven (NL); MENSER, Bernd, 5656AG Eindhoven (NL); VOGTMEIER, Gereon, Eindhoven (NL); FORTHMANN, Peter, Eindhoven (NL); WEIß, Steffen, Eindhoven (NL); HELLE, Michael Günter, Eindhoven (NL)
(74) Representative: Philips Intellectual Property & Standards

(57) **Abstract**

An image processing system (100) and related method. The image processing system comprises an input interface (102) configured to receive X-ray image data of an object acquired by an X-ray imaging apparatus during a scan and to receive camera image data of the object acquired by a camera system having a field of view of the object. A geometry determiner (104) of the system is configured to analyze the camera image data of the object to determine geometry information of the object and distance information of the object to a detector of the X-ray imaging apparatus. A scatter estimation module (106) of the system is configured to estimate a scatter profile of scatter present in the X-ray image data by use of the determined geometry information of the object and the determined distance information of the object to the detector of the X-ray imaging apparatus.

## Description

### FIELD OF THE INVENTION

The invention relates to an image processing system, to an image processing method, and to a computer program element.

### BACKGROUND OF THE INVENTION

Scatter is an integral problem in diagnostic X-ray imaging. Scatter may be reduced by scatter grids, however, alternatively, software-based scatter correction can be applied when using no scatter grids or in combination with scatter grids. A disadvantage of grids is the fact that they do not only reduce scatter, but also part of the primary radiation. Especially when used with mobile detectors, a misaligned grid can significantly reduce primary radiation and can cause additional shading artefacts. Another drawback of grids in mobile applications is the additional weight of the detector. In fixed detector systems, a moving grid increases the cost of material due to the additional mechanics.

Software based scatter correction methods are either based on line-integrals estimated derived from the two-dimensional (2D) images, or based on a first pass three-dimensional (3D) reconstruction in case of 3D method that are applicable in volume imaging. An example of a 2D kernel-based scatter estimation method is described in WO 2007/148263 A1.

### SUMMARY OF THE INVENTION

There may be a need in the art to further refine or improve X-ray scatter correction.

The object of the present invention is solved by the subject matter of the independent claims where further embodiments are incorporated in the dependent claims. It should be noted that the following described aspect of the invention equally applies to the image processing system, to the image processing method, and to the computer program element.

According to a first aspect of the present invention, there is provided an image processing system, comprising:
an input interface configured to receive X-ray image data of an object acquired by an X-ray imaging apparatus during a scan and to receive camera image data of the object acquired by a camera system having a field of view of the object;
a geometry determiner configured to analyze the camera image data of the object to determine geometry information of the object and distance information of the object to a detector of the X-ray imaging apparatus; and
a scatter estimation module configured to estimate a scatter profile of scatter present in the X-ray image data by use of the determined geometry information of the object and the determined distance information of the object to the detector of the X-ray imaging apparatus.

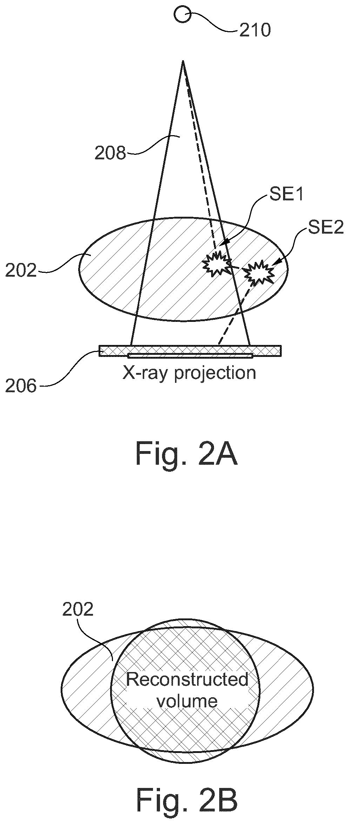

Current scatter estimation methods use the measured detector data (in case of projection-based 2D methods) or a first pass reconstruction (in case of image-based 3D methods). In both approaches, the scattering objects may exceed the measurement field. It may extend the detector and from the volume of the patient not imaged by the detector photons may scatter back into the detector elements. An exemplary scenario is shown in FIG. 2A. This problem may be more severe for 3D image-based scatter estimation methods, which may suffer from the limited field-of-view of the reconstruction. An exemplary scenario is shown in FIG. 2B.

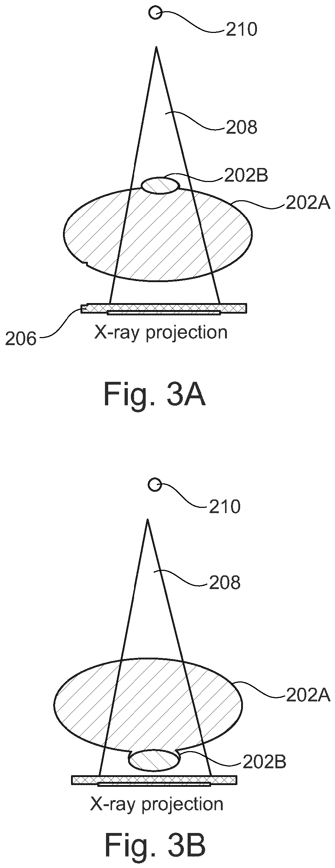

The inventors of the present application have also found out that the 2D projection data may comprise projection data of two or more objects, such as a patient and a device that is attached to the patient (e.g., orthosis, tube, cables, and the like) and/or other objects in the field of view, like patient table, head rest, and the like. However, the arrangement of the two or more objects and their distances to the X-ray detector cannot be derived from the 2D projection data, which however may have a big impact on the scatter estimation. An exemplary scenario is shown in FIGS. 3A and 3B.

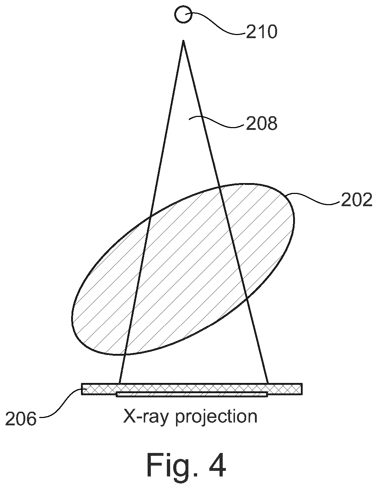

The inventors of the present application have further found out that the object may be tilted at an angle to the X-ray detector, which may result in uncertainty in determining the patient shape and position from the 2D projection data. An exemplary scenario is shown in FIG. 4.

In order to address one or more of the above problems, the image processing system is proposed to obtain camera image data of the object that is acquired by a camera system. The volume of the patient is seen by the camera system that has a field of view covering the patient, but not by the X-ray detector. The integration of cameras into diagnostic X-ray systems for patient positioning offer the opportunity to derive additional information from the video images to achieve a more accurate software-based scatter correction. The additional information comprises geometry information of the object and distance information of the object to the X-ray detector. Additionally, the camera image data may be used to identify and distinguish different objects, such as the patient and the device attached to the patient. It is possible to identify them in the camera image data using e.g., objection detection approaches, and to derive their distance to the X-ray detector. Accordingly, the scatter estimation may be further improved in the scenario of two or more objects. Further, as noted above, there is uncertainty in the existing projection-based scatter estimation method, e.g. due to the projection of the scene in the X-ray image, the depth component is lost. This uncertainty may be reduced when cameras provide information on the shape and position of the object.

In some radiographic procedures at least two X-ray images along different direction are acquired, such as thorax imaging including a frontal image and a lateral image, or a mammogram X-ray including a head-feet image and a mediolateral oblique image. In such cases, the image processing system as disclosed herein may use both information in the scatter correction by utilizing the camera image information from both directions.

In embodiments, the geometry determiner is configured to fit an object model to the object in the camera image data to determine the geometry information of the object and the distance information of the object to the detector, wherein the object model comprises one or more geometric parameters describing a geometry of the object. Accordingly, the information may be combined with a generic patient model which is fit to the video images of the scene. If a object model, such as patient model, is to be used, the model can be picked based on the camera data, to best fit e.g., the patient's anatomy.

In embodiments, the object model comprises a patient model comprising annotated data with tissue information. This may allow obtaining still more accurate scatter correction, in particular, for objects with heterogeneous density distribution which may be caused by the presence of bones in the imaged anatomy. More specifically, while the mass attenuation coefficients for photoelectric effect and Compton scatter of soft tissue are very similar to the coefficients of water, X-ray absorption and scattering properties of bone are very different. In other words, for the same primary absorption, scatter from a water object is larger than the scatter from a bone object. The tissue determiner allows forming scatter kernels that are adapted to the local material composition to so account for the different scattering properties of, in particular, bone and soft tissue.

In embodiments, the patient model is adapted according to patient data. Additional information can be used to fine-tune the patient model when processing other available data such as patient weight, water-fat-fraction, information about implants etc. Such information can be retrieved e.g. from electronic medical records but can also be measured as part of the patient preparation.

In embodiments, the patient data comprises one or more of the following data:
patient weight, water-fat-fraction, and information about an implant.

In embodiments, the geometry determiner is configured to analyze the camera image data of the object to determine a presence of an additional X-ray scattering object and in response to determining the presence of the additional X-ray scattering object, to determine distance information of the device to the detector of the X-ray imaging apparatus. This may allow to determine the presence of additional X-ray scattering objects. As noted above, when two or more X-ray scattering objects are present, their distance to the X-ray detector cannot be derived from 2D projection data. Thus, the camera image data may be used to identify and distinguish different X-ray scattering devices and to determine their distance to the X-ray detector.

In embodiments, the additional X-ray scattering object comprises one or more of the following: a device that is attached to a patient, and an additional X-ray scattering object that supports and/or fixates an anatomy of a patient. For known devices, it is possible to use a database of scatter distribution from which the data associated with the known devices can be obtained.

In embodiments, the scatter estimation module is configured to estimate the scatter profile of scatter present in the X-ray image data by use of Monte-Carlo simulations.

In embodiments, the scatter estimation module is configured to estimate the scatter profile of scatter present in the X-ray image data by use of a kernel-based scatter estimation approach.

In embodiments, the scatter estimation module is configured to estimate the scatter profile of scatter present in the X-ray image data by use of a neural network, wherein the neural network has been trained to reproduce the scatter profile given a function of the X-ray image data and the determined geometry information of the object and the determined distance information of the object to the detector of the X-ray imaging apparatus. In some embodiments, the geometry determiner and the scatter estimation module may be two separate modules. In some embodiments, the geometry determiner and the scatter estimation module may be implemented as a single module. For example, the complete camera image is fed into the neural network as side information to generate the scatter profile. Additionally, in the training data, the scatter profile may comprise a scatter profile generated in various ways. In some examples, the scatter profile in the training data may comprise an output of Monte-Carlo simulations. In some examples, the scatter profile in the training data may comprise an output of scatter kernel estimations. In some other examples, the scatter profile in the training data may comprise object measurements acquired with and without a scatter grid. In some further examples, the scatter profile in the training data may comprise a combination of two or more of the above-described scatter profiles.

In embodiments, the scatter estimation module is configured to estimate the scatter profile of scatter present in the X-ray image data by performing a first pass reconstruction and estimating the scatter profile from the first pass reconstruction by use of the determined geometry information of the object and the determined distance information of the object to the detector of the X-ray imaging apparatus, wherein the estimated scatter profile is usable to for scatter compensation of the X-ray image data before a second pass reconstruction is performed. The limited field of view (FOV) is an issue in 3D scatter estimation methods, and the camera data can be used to extend the patient and other objects beyond the FOV.

In embodiments, the image processing system further comprises an exposure determination module configured to estimate a scatter profile from the camera image data prior to the scan, and to determine one or more of a kilovoltage, a milliamperage, and an exposure time based on the scatter profile estimated from the camera image data. Accordingly, part of the scatter correction calculations can be done before exposure. Automatic exposure control could be more precise due to existing scatter information before doing the scan. This may save dose and/or optimize dose for best image quality

In embodiments, the image processing system further comprises a scatter correction module configured to perform scatter correction based on the scatter profile of scatter present in the X-ray image data.

According to a second aspect of the present invention, there is provided an image processing method, comprising:
receiving X-ray image data of an object acquired by an X-ray imaging apparatus during a scan and to receive camera image data of the object acquired by a camera system having a field of view of the object;
analyzing the camera image data of the object to determine geometry information of the object and distance information of the object to a detector of the X-ray imaging apparatus; and
estimating a scatter profile of scatter present in the X-ray image data by use of the determined geometry information of the object and distance information of the object to the detector of the X-ray imaging apparatus.

According to a third aspect of the present invention, there is provided a computer program element, which, when being executed by at least one processing unit, is adapted to cause the processing unit to perform the method according to the second aspect.

According to a fourth aspect, there is provided a computer readable medium having stored thereon the program element.

The input imagery may be drawn from projection domain but application of the proposed system and method to reconstructed imagery is not excluded herein. Scatter correction in image domain may be done performing first reconstruction from projection imagery, with a less accurate scatter correction. The scatter is then estimated as described above, but this time based on the reconstructed image.

Although the present disclosure is mainly concerned with medical X-ray imaging, non-medical applications are not excluded herein, such as baggage screening, or non-destructive material testing.

References in this disclosure to "1D", "2D" or "3D" are shorthand for spatial one-, two- or three-dimensionality, respectively.

### BRIEF DESCRIPTION OF THE DRAWINGS

Exemplary embodiments of the invention will now be described with reference to the following drawings, which are not to scale, wherein:
Fig. 1 illustrates a schematic block diagram of an imaging arrangement;
Fig. 2A illustrates a truncation issue of 2D scatter estimation;
Fig. 2B illustrates a truncation issue of 3D scatter estimation;
Figs. 3A and 3B illustrate an arrangement issue of multiple objects on the scatter estimation;
Fig. 4 illustrates an arrangement issue of a single object on the scatter estimation;
Fig. 5 illustrates an exemplary image processing system;
Fig. 6 illustrate another exemplary image processing system;
Fig. 7 illustrates a further exemplary image processing system; and
Fig. 8 illustrates an exemplary flow chart describing an image processing method.

### DETAILED DESCRIPTION OF EMBODIMENTS

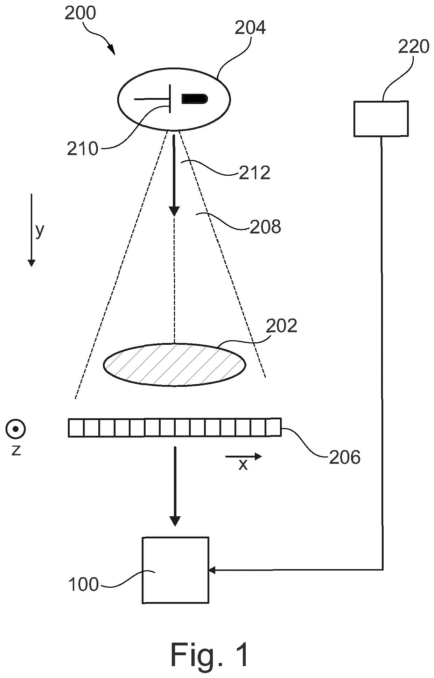

Fig. 1 illustrates a schematic diagram of an imaging processing system 100 and an X-ray imaging apparatus 200.

The X-ray imaging apparatus 200 is shown in a schematic side elevation view in Fig. 1. Embodiments of the X-ray imaging apparatus 200 may include, but are not limited to, a C-arm imaging apparatus, a CBCT (Cone Beam CT) scanner, a CT scanner, a mammography apparatus, a so-called O-arm device, a robotic C-arm system e.g., with two robotic arms, one carrying the tube and the other the detector, or a radiography apparatus, or other, configured to acquire an X-ray image of an object 202.

The object 202 is shown in cross-section with an imaging axis z. In Fig. 1, the imaging axis z extends into the drawing plane. Although the main applications for the X-ray imager envisaged herein are in the medical field, non-medical contexts such as non-destructive material testing or baggage screening, etc. are not excluded herein. Accordingly, the term "object" is used herein in the general sense to include animate "objects" such as a human or animal patient, or anatomic parts thereof but also includes inanimate objects.

In the illustrated example, the X-ray imaging apparatus 200 includes an X-ray source 204 and an X-ray sensitive detector 206. In use, the object 202 is positioned along axis z in an examination region between the X-ray source 204 and the X-ray detector 206. The X-ray source 204 is energized to produce an X-ray beam 208 which emanates from a focal spot 210 and traverses the examination region and hence at least a region of interest of the object 202. In Fig. 1, the optical axis is indicated by an arrow 212, and the main propagation direction of the X-ray beam, with axis y parallel to the optical axis 212. Specifically, the optical axis 212 runs from the focal spot 210 to the X-ray detector 206 and intersects the X-ray detector 206 at a point. The image surface has coordinates x,z, with x being orthogonal to imaging axis z, and both, *x,z,* being orthogonal to the optical axis 212 or axis y.

The X-radiation interacts with matter (e.g., tissue, bones, etc.) of the object 202. After interaction, the X-radiation emerges at the far side of object 202 to then impinge on the X-ray detector 206. The impinging X-radiation is detected by the X-ray detector 206 and converted into electrical signals. The electrical signals are converted by suitable conversion circuitry (not shown) into image values which may then be processed into X-ray images by an image processor. The image values may be arranged in rows and/or columns which form the X-ray image. Suitable imaging software may be used to visualize the imagery on one or more display devices. The images may also be stored or otherwise processed. The X-ray images are capable of revealing details of the internals of the imaged object 202. This can help in diagnosis and therapy or other examination of the imaged object 202.

The X-ray image provided by the X-ray imaging apparatus 200 is either a projection image or reconstructed imagery. Traditional radiography is projection image-based. Reconstructed imagery emerges for instance in the context of CT or C-arm imaging. In which case the signal processing chain includes a re-constructor that runs a re-construction algorithm such as filtered back-projection or other. The re-constructor processes the projection imagery which is located in the plane of the detector and converts the projection imagery into one or more slice images that are located in the image domain or the examination region in the plane perpendicular to that of the detector plane. The re-constructor hence maps projection imagery from the projection domain into slice imagery in the image domain. It will be understood that in general multiple projection images are acquired from different directions of the objects. In CT this is achieved by a rotational arrangement where the X-ray source and/or the detector rotates around the object 202 to acquire this set of multiple projection imagery along different directions. It is this set of projection imagery that is then fed into the re-constructor to obtain one or more slice images which can be thought of as making up together an image volume that is an image approximation of the examination region that includes the object 202.

The X-ray detector 206 may be a flat panel detector, but curved embodiments are also envisaged herein. The detector 206 may be of the direct conversion type or of the indirect conversion type.

Fig. 1 may illustrate an exemplary radiographic procedure where X-ray images along a single direction are acquired. It will be appreciated that in some other exemplary radiographic procedures (not shown), at least two X-ray images along different direction are acquired, such as thorax imaging including a frontal image and a lateral image, or a mammogram X-ray including a head-feet image and a mediolateral oblique image. In such cases, the image processing system as disclosed herein may use both information's in the scatter correction by utilizing the camera image information from both directions.

The X-radiation beam 208 emanating from the focal spot 210 may be thought of as a diverging bundle of individual pencil beams that propagate along a set of respective imaginary geometrical rays that can be cast from the focal spot 210 to the different detector pixel positions on the detector 206. X-ray photons travel along respective ones of the geometrical rays to form those pencil beams. The photons, prior to impacting with matter, have an initial energy when they emerge from the focal spot 210 of the X-ray source 204. Some photons travelling along a given geometrical ray are absorbed thus reducing the intensity of the X-radiation seen at the respective detector pixel located at the end of the respective geometrical ray. However, not all of the reduced intensity can be attributed to absorption events. Other photons in the given pencil beam may get scattered, so diverge off their intended path along the geometrical ray as shown in Fig. 2A. Because of one or more scattering events SEs, such as scattering events SE1 and SE2, the photon diverges off the intended path and is instead incident at another detector pixel in relatively close neighborhood of the intended pixel. Therefore, a certain amount of intensity that should have been seen at the intended pixel is instead registered at another pixel location as a scattered photon. In the example shown in Fig. 2A, when multiple scattering events are taken into account, the total size of the body is crucial. It may extend the detector and from the volume of the patient not imaged by the detector photons may scatter back into the detector elements.

Fig. 2B illustrates an exemplary 3D scatter estimation algorithm that operates on the object 202. This approach may perform a first pass reconstruction and then estimate the scatter component of the projection data by performing X-ray transport calculations through the object derived from the first pass reconstruction. The computed scatter signal is then subtracted from the original projection data before a second pass reconstruction is performed. However, as shown in Fig. 2B, the existing methods may suffer from the limited field-of-view of the reconstruction.

Fig. 3A and Fig. 3B illustrate an exemplary scenario where the X-ray beam passes two objects including a first object 202A and a second object 202B. For example, the first object 202A may be a patient, and the second object 202B may be an object (e.g., orthosis) that is attached to the surface of the patient and that is visible to the camera. Fig. 3A and Fig. 3B show similar objects 202A and 202B except for their spatial arrangement. In some other scenarios (not shown), the first object 202A may be a patient, and the second object 202B may be a prosthesis or implant which may be inside the patient or have parts visible at the surface of the patient. In both scenarios, the distance of the first object 202A and the second object 202B to the X-ray detector cannot be derived from the 2D projection data, which may have a big impact on the scatter estimation.

Fig. 4 illustrates an exemplary scenario where the object 202 is tilted at angle to the X-ray detector 206. However, this cannot be derived from the 2D projection data. This may result in uncertainty in determining the patient shape and position from the 2D projection data.

To address one or more problems as shown in Figs. 2A to Fig. 4, the image processing system 100 is proposed to obtain camera image data of the object acquired by a camera system having a field of view of the object. For example, as shown in Fig. 1, a camera system 220 is provided that comprises one or more sensors. The image processing system 100 may be coupled to the camera system 220 via a wired or wireless connection. In some examples, the one or more sensors may comprise at least one camera that captures a 2D image of the patient 202 by exposure of non-ionizing radiation (e.g., visible, near infrared and/or infrared light) or sound (e.g., acoustic sound and/or ultrasound). In some examples, the one or more sensors may include at least one range camera that is configured to acquire 3D image data of patient 202 by exposure of non-ionizing radiation or sound. The 3D image data is an array of pixels each pixel having a position in said array and a value. Each pixel position corresponds to a position of a point on the patient's surface and the value is directly related to or can be expressed in terms of the distance between camera's sensor and said patient surface point. Pixel values vary with sensor-object distance. Point cloud representation of the 3D image data set is also envisaged. In other words, the 3D image data "follows" or describes the outer surface or perimeter of the patient 202 in 3D space. In some examples, the camera system may comprise a sensor that is arranged at the housing of the X-ray source 204 by way of example. It will be appreciated that the one or more sensors may be arranged at any suitable position. For example, the camera system 220 may comprise at least one sensor arranged on the ceiling, on the overhead carriage, on the wall, or on the wall-stand. In some implementations, at least one sensor may be positioned to capture essentially the entirety of the examination region or at least that portion of the examination region where the patient 202 can be expected to reside during the image acquisition. While only one camera is illustrated in Fig. 1, it will be appreciated that the camera system 220 may comprise a single 2D camera, multiple 2D cameras, a 3D camera, or multiple 3D cameras.

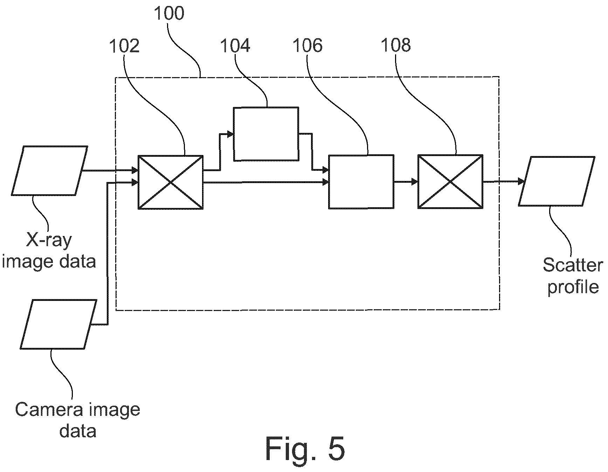

A schematic block diagram of the proposed processing system 100 is shown in Fig. 5. The image processing system 100 may be arranged to process projection imagery as received from the X-ray detector. Alternatively, the image processing system 100 may also act on reconstructed imagery. The image processing system 100 may be implemented in numerous ways (e.g., such as with dedicated hardware) to perform the functions described herein. A "processor" is one example of the image processing system 100, which employs one or more microprocessors that may be programmed using software (e.g., microcode) to perform various functions described herein. The image processing system 100 may be implemented with or without employing a processor, and also may be implemented as a combination of dedicated hardware to perform some functions and a processor (e.g., one or more programmed microprocessors and associated circuitry) to perform other functions. Examples of the image processing system that may be employed in various embodiments of the present disclosure include, but are not limited to, conventional microprocessors, application specific integrated circuits (ASICs), and field-programmable gate arrays (FPGAs). In various implementations, a processor may be associated with one or more storage media (generically referred to herein as "memory," e.g., volatile and non-volatile computer memory). In some implementations, the storage media may be encoded with one or more programs that, when executed on one or more processors and/or controllers, perform at least some of the functions described herein. Various storage media may be fixed within the computing device or may be transportable, such that the one or more programs stored thereon can be loaded into the computing device so as to implement various aspects of the present disclosure described herein. The terms "program" or "computer program" are used herein in a generic sense to refer to any type of computer code (e.g., software or microcode) that can be employed to program one or more processors. As an example, the image processing system 100 or some of its components may reside in an operator console running as software routines. The components may be programmed in a suitable scientific computing platform such as Matlab^{®} or Simulink^{®} and then translated into C++ or C routines maintained in a library and linked when called on by the operator console. The components of the image processing system may be implemented as software modules or routines in a single software suit and run on a general purpose computing unit such as a workstation associated with the imager or a server computer associated with a group of imagers. Alternatively, the components of the image processing system IPS may be arranged in a distributed architecture and connected in a suitable communication network. The image processing system may be fully integrated into the X-ray imaging apparatus.

Either automatically or upon request by an operator, the image processing system 100 is configured to receive, via an input interface 102, X-ray image data of an object acquired by an X-ray imaging apparatus during a scan and to receive camera image data of the object acquired by a camera system having a field of view of the object. For example, the X-ray image data may be acquired by the X-ray imaging apparatus 200 and the camera image data may be acquired by the camera system 220 shown in Fig. 1. In the illustrated example, only a single input interface is illustrated. It will be appreciated that in other embodiments, the input interface may comprise two separate interfaces including an X-ray image data interface and a camera interface. The camera interface may provide a camera interface functionality for different types of actual but also future cameras that can deliver relevant information but with more precise data depending on the type of camera and the camera complexity. The parameterized data from the interface is used for the scatter correction algorithm and depending on the availability of data and the precision of data the scatter algorithm may be selected and/or adapted in a flexible way. The camera interface may be open for the connection of more than one camera which could be beneficial to get geometry and distance information for different patient positions but also geometry information from different angles, such as front view, and side view. The input interface 102 may be e.g., a USB interface, a Wi-Fi interface, an Ethernet interface, and the like.

The camera image data of the object is then forwarded to the geometry determiner 104, which is configured to analyze the camera image data of the object to determine geometry information of the object and distance information of the object to a detector of the X-ray imaging apparatus. The geometry information of the object may comprise a set of angular or rectangular coordinate positions that describe a shape and a spatial position of the object. The distance information of the object to the detector may comprise a relative position between the object and the detector.

The geometry determiner 104 may determine the geometry information of the object and distance information of the object to the X-ray detector in several ways. In some examples, one or more markers may be attached to the object 202 e.g., to mark a landmark or an anatomy of the object. The camera system 220 may capture an image of the object to detect the position of the one or more markers and to determine the overall body height, and overall body width of the patient accordingly. In some other examples, no markers need be applied to the object during the image acquisition. For example, the patient may walk into the examination room "as is" and towards a desired target spot therein. The camera system 220 may capture 3D image data of the patient 202 showing 3D shape and geometry of the patient's 3D contours. Based on such information, a set of geometry parameters may be determined that includes the geometry information of the object and distance information of the object to the X-ray detector.

In some further examples, the geometry determiner 104 may be configured to fit an object model to the object in the camera image data to determine the geometry information of the object and the distance information of the object to the detector. The object model comprises one or more geometric parameters describing the geometry of the object. In one example, the object model may comprise a patient model and the geometry determiner 104 may adapt the patient model to a particular patient by using a set of non-rigid registration parameters. The patient model may be a generic representation of a surface of a human. The patient model may be not specific to any patient or is specific to a patient meeting a norm. The patient model includes one or more internal organs and/or one or more tissues. The patient model may be an annotated 3D model with annotated data comprising tissue information, which is used to obtain a radiation specific weighting factor and a tissue specific weighting factor of at least one internal organ and/or at least one type of tissues within the patient model. In some examples, the tissue information may comprise an organ identifier used to identify at least one organ within the patient model and/or a tissue identifier used to identify a tissue type of at least one within the patient model. For example, if the internal organ (e.g., liver) comprises a single type of tissues (e.g., liver), the organ identifier may be used to retrieve the tissue specific absorption coefficients and weighting factors from a database for the calculation of a local effective dose. In some examples, the tissue information may comprise the absorption coefficient and the tissue weighting factors. This may be beneficial if the internal organ (e.g., bone marrow) comprises two or more types of tissues. In such cases, the tissue information may comprise an attenuation coefficient of at least one tissue type of tissues and a tissue specific weighting factor of at least one tissue type of the tissues within the patient model. Additional information may be used to fine-tune the patient model when processing other available data such as patient weight, water-fat-fraction, information about implants etc. Such information may be retrieved e.g. from electronic medical records but can also be measured as part of the patient preparation.

In some examples, there may be one or more additional X-ray scattering objects in addition to the patient. For example, there may be one or more devices attached to the patient which are inside the field of view of the X-rays . The one or more devices may comprise a device that is not inside the patient, such as a device attached to the surface of the patient. Examples of such devices may include, but are not limited to, orthosis, tube, cables, and the like. Further examples may include an additional X-ray scattering object that supports and/or fixates an anatomy of the patients, e.g., a pillow, a patient table, a head read, other objects that support the patient to be in the imaging pose.

The one or more devices may comprise a device that is only partially inside the patient but the 3D shape of the device is known. The one or more devices may comprise a device that is only partially visible in the camera image, but the 3D shape of the device is known or can be estimated from the camera image. In the above cases, the distance of the patient and the device (e.g., cables, orthosis, etc.) to the X-ray detector cannot be derived from the 2D projection data. The geometry determiner 104 as disclosed herein may be configured to analyze the camera image data of the object to determine a presence of an additional X-ray scattering object and in response to determining the presence of the additional X-ray scattering device, to determine distance information of the additional X-ray scattering device to the detector of the X-ray imaging apparatus. For example, the geometry determiner 104 may apply an object detection algorithm to detect the presence of an additional X-ray scattering device. Exemplary objection detection algorithms may include convolutional neural networks (R-CNN, Region-Based Convolutional Neural Networks), Fast R-CNN, and YOLO (You Only Look Once). The objection detection algorithms are not limited to neural network based approaches, but could also include classical methods like template matching or feature, color, and classifier-based approaches. In response to the detection of the presence of the device, the geometry determiner 104 may perform image segmentation and determine the distance of the device to the X-ray detector. In some examples, the additional X-ray scattering object may be a device that is only partially visible in the camera image, but the 3D shape of the device is known. In these examples, the geometry determiner 104 may apply an object detection algorithm to identify the additional X-ray scattering object, and to determine the geometry of the additional X-ray scattering object based on the known 3D shape of the device. In some further examples, the additional X-ray scattering object may be a device that is only partially visible in the camera image, but the 3D shape of the device is not known. In these examples, the geometry determiner may perform a joint analysis of X-ray and camera image to determine the geometry information of the object and distance information of the additional X-ray scattering object to the detector. Such information may be added to the scatter estimation module to for a better scatter estimation.

The results output by the geometry determiner 104 are then forwarded to the scatter estimation module 106 together with the received X-ray image data, which is configured to estimate a scatter profile of scatter present in the X-ray image data by use of the determined geometry information of the object and distance information of the object to the detector of the X-ray imaging apparatus.

The scatter estimation module 106 may apply different approaches to estimate the scatter profile of the scatter present in the X-ray image.

In an example, the scatter estimation module 106 may be configured to estimate the scatter profile of scatter present in the X-ray image data by use of Monte-Carlo simulations. Monte-Carlo simulations have the ability to estimate the expected number of X-ray photons passing through the patient, energy deposited leading to the patient dose, and the number of X-rays absorbed by the image detector. Monte-Carlo models that take into consideration the operational parameters, and geometry can effectively determine the characteristics of scattered X-rays. Therefore, given additional information from the camera image data, additional parameters determined by the geometry determiner can be added to the Monte-Carlo simulations. These additional parameters include the determined geometry information of the object and the determined distance information of the object to the detector of the X-ray imaging apparatus. Optionally, the additional parameters may include material probabilities at given position, such as bone, soft tissue, water predicted from a patient model comprising annotated data with tissue information. As a further option, if there is an additional X-ray scattering object, the additional parameters may include the distance of the additional X-ray scattering object to the X-ray detector. Examples of the additional X-ray scattering object may include orthosis, tube, cables, and the like.

In an example, the scatter estimation module 106 may be configured to estimate the scatter profile of scatter present in the X-ray image data by use of a kernel-based scatter estimation approach. The kernel-based scatter estimation approach may make use of precalculated needle-beam Monte-Carlo simulations of primitive geometries such as cuboids or ellipsoids with vary dimensions. To estimate scatter within a measured projection, one of the precalculated needle-beam kernels is assigned to every detector pixel according to an appropriate similarity metric. Finally, all kernels are summed up including correction terms that account for differences between the prior object shape and the actual patient shape. An example of the kernel-based scatter estimation method is described in WO 2007/148263 A1. The existing approach simulates scatter radiation and parameterizes the results that just uses attention from the X-ray image. The scatter estimation module 106 as described herein may add the camera derived information to the kernel-based scatter estimation module by modifying the height and width of the primitive geometries such as cuboids or ellipsoids delivering scatter estimates, changing the mean density of the material inside the primitive geometries, providing asymmetric shape of the primitive geometries, and/or changing the distance of the primitive geometries to the X-ray detector. The camera derived information may include the determined geometry information of the object and the determined distance information of the object to the detector of the X-ray imaging apparatus. Optionally, the camera derived information may include material probabilities at given position, such as bone, soft tissue, water predicted from a patient model comprising annotated data with tissue information. As a further option, if there is an additional X-ray scattering object, the additional parameters may include the distance of the additional X-ray scattering object to the X-ray detector.

In an example, the scatter estimation module 106 may be configured to estimate the scatter profile of scatter present in the X-ray image data by use of a neural network. The neural network has been trained to reproduce the scatter profile given a function of the X-ray image data and the determined geometry information of the object and the determined distance information of the object to the detector of the X-ray imaging apparatus. In the training data, the scatter profile may comprise a scatter profile generated in various ways. In some examples, the scatter profile in the training data may comprise an output of Monte-Carlo simulations. In some examples, the scatter profile in the training data may comprise an output of scatter kernel estimations. In some other examples, the scatter profile in the training data may comprise object measurements acquired with and without a scatter grid. The existing algorithm, such as the neural network described in Joscha Maier et al: Real-time scatter estimation for medical CT using the deep scatter estimation: Method and robustness analysis with respect to different anatomies, dose levels, tube voltages, and data truncation. Med Phys. 2019; 46(1); 238-249, uses a neural network that has been trained to produce the output of Monte-Carlo simulations given a function of the X-ray projection data. Given additional information from the camera image, additional parameters, such as determined geometry information of the object and the determined distance information of the object to the detector of the X-ray imaging apparatus, may be provided as a further input to the neural network for a more accurate scatter prediction. The additional parameters may further comprise information like material probabilities at given position, and distance of the additional X-ray scattering object to the X-ray detector.

In an example, the camera derived information may also be used to support image-based scatter correction methods in 3D imaging applications. The existing scatter estimation method in 3D imaging may perform a first pass reconstruction and then estimate the scatter component of the projection data by performing X-ray transport calculations through the object derived from the first pass reconstruction. The computed scatter signal is then subtracted from the original projection data before a second pass reconstruction is performed. However, as discussed with respect to Fig. 2B, the existing method may suffer from the limited field-of-view of the reconstruction. In order to solve this problem, the scatter estimation module 106 may be configured to estimate the scatter profile of scatter present in the X-ray image data by performing a first pass reconstruction and estimating the scatter profile from the first pass reconstruction by use of the determined geometry information of the object and the determined distance information of the object to the detector of the X-ray imaging apparatus. Accordingly, the extended field-of-view of the camera can be used to spatially extend the truncated first pass reconstruction used to calculate scatter. The estimated scatter profile is usable to for scatter compensation of the X-ray image data before a second pass reconstruction is performed.

The scatter profile is then forwarded to an output interface 108, which is configured to output the scatter profile to e.g., a scatter correction device (not shown) for further processing or to a storage device (not shown) via a wired or wireless connection. The output interface may be a USB interface, a Wi-Fi interface, an Ethernet interface, and the like.

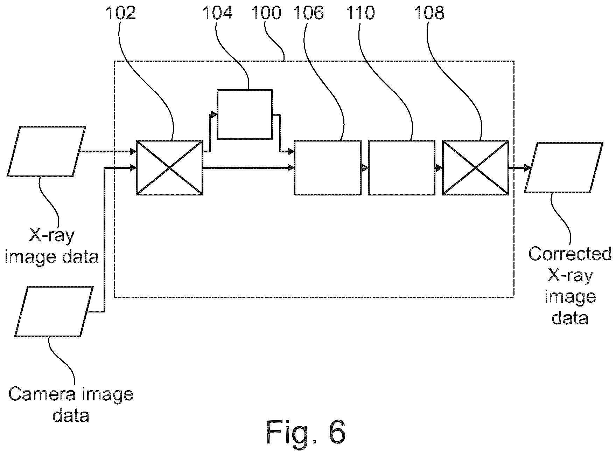

In some embodiments, the scatter correction device may be implemented as a component of the image processing system 100. For example, Fig. 6 illustrates a further exemplary imaging processing system 100, in which the scatter correction device is implemented as a scatter correction module 110, which is configured to perform scatter correction based on the scatter profile of scatter present in the X-ray image data. The corrected X-ray image data is then forwarded to the output interface 108, which is configured to output the corrected X-ray image data.

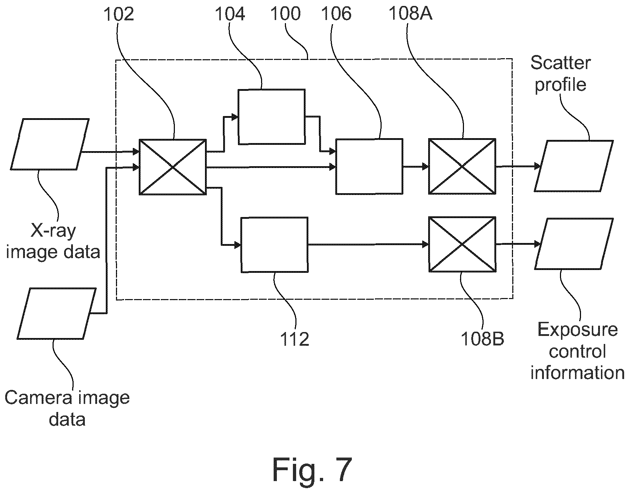

In some embodiments, as shown in Fig. 7, the image processing device 100 may additionally comprise an exposure determination module 112 configured to estimate a scatter profile from the camera image data prior to the scan, and to determine one or more of a kilovoltage, a milliamperage, and an exposure time based on the scatter profile estimated from the camera image data. In other words, the exposure determination module 112 estimates scatter from the camera image alone prior to the scan. In this case it could be used to also affect exposure control. In this way, automatic exposure control could be more precise due to existing scatter information before doing the scan. It is also possible to predict the scatter profile and improve image quality for the whole image besides a small area of reference detector. This may save dose and/or optimize dose for enhanced image quality. The exposure control information may be output, via the output interface 108, to the X-ray imaging apparatus 200 shown in Fig. 1. In the exemplary image processing system 100 shown in Fig. 7, the scatter profile and the exposure control information are output via two different output interfaces, i.e., first output interface 108A and second output interface 108B. In some other examples (not shown), the scatter profile and the exposure control information may be output via the same output interface.

Figs. 6-8 may show the geometry determiner and the scatter estimation module as two separate modules by way of example. It will be appreciated that in some other embodiments, the geometry determiner and the scatter estimation module may be implemented as a single module. For example, the complete camera image is fed into the neural network as side information to generate the scatter profile.

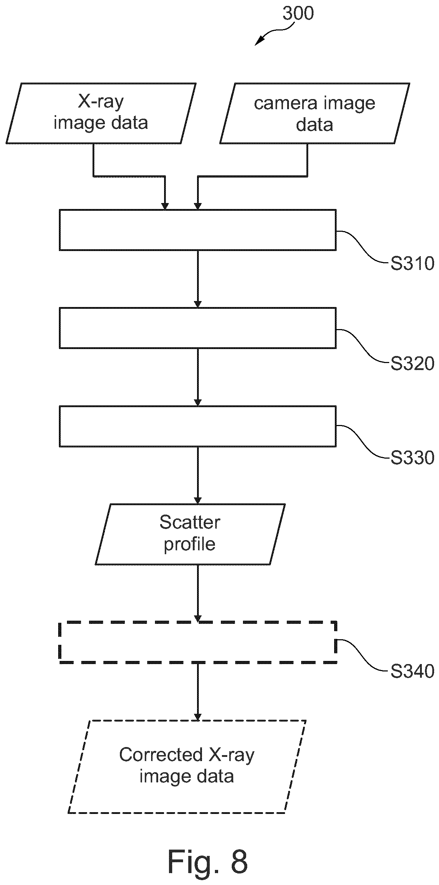

Reference is now made to a flow chart in Fig. 8 to describe a method 300 of image processing as implemented by the image processing system. However, it will be understood that the steps described in the following are not necessarily tied to the architecture of the image processing system as described above but the following steps may also be understood as a teaching in its own right.

At step S310, X-ray image data of an object to be scatter-corrected is received. Additionally, camera image data of the object is received.

The X-ray image data of the object may be retrieved from an image database, such as a PACS (picture archiving and communication system) in a HIS (hospital information system). Alternatively, the input X-ray image data may be supplied directly from the X-ray imaging apparatus. The X-ray image data of the object to be scatter-processed may be received wirelessly or through a wired connection. The input X-ray image data is either a projection image from projection domain or may be a reconstructed image in imaging domain, reconstructed by a reconstruction algorithm from projection imagery. If the X-ray image data is a reconstructed one, the scatter correction is preferably still applied to the projection imagery. The X-ray image data may be 2D or 3D. The X-ray image is acquired of an object, such as a region of interest of a human or animal subject, such as a skull, chest or heart, or other. In some instances, a suitable contrast agent is injected beforehand to increase contrast for soft tissue.

The camera image data of the object may be retrieved from an image database, such as a PACS in a HIS that stores the X-ray image data together with the camera image data of the object during a scan. Alternatively, the input camera image data may be supplied directly from the camera system. The camera image data of the object may be received wirelessly or through a wired connection. In some examples, the camera image data may be acquired by at least one camera that captures a two-dimensional image of the object by exposure of non-ionizing radiation or sound. The camera system may comprise at least one camera that is arranged at the housing of the X-ray source by way of example. It will be appreciated that the camera may be arranged at any suitable position. For example, the camera system may comprise a camera arranged on the ceiling, on the overhead carriage, on the wall, or on the wall-stand. In some implementations, the camera may be positioned to capture essentially the entirety of the examination region or at least that portion of the examination region where the object can be expected to reside during the image acquisition.

The proposed method adds camera derived information to the scatter estimation method to achieve a more accurate software-based scatter correction.

To this end, in step S320, the camera image data of the object is analyzed to determine geometry information of the object and distance information of the object to a detector of the X-ray imaging apparatus. The geometry information of the object and distance information of the object to the X-ray detector may be determined in several ways.

In some examples, one or more markers may be attached to the object e.g., to mark a landmark and/or an anatomy of the object. The camera system may capture an image of the object to detect the position of the one or more markers and to determine the overall body height, and overall body width of the patient accordingly.

In some other examples, no markers need be applied to the object during the image acquisition. For example, the patient may walk into the examination room "as is" and towards a desired target spot therein. The camera system may capture 3D image data of the patient showing 3D shape and geometry of the patient's 3D contours. Based on such information, a set of geometry parameters may be determined that includes the geometry information of the object and distance information of the object to the X-ray detector.

In some further examples, an object model may be fitted to the object in the camera image data to determine the geometry information of the object and the distance information of the object to the detector. The object model comprises one or more geometric parameters describing the geometry of the object. In one example, the object model may comprise a patient model and the patient model may be adapted to a particular patient by using a set of non-rigid registration parameters. The patient model may be a generic representation of a surface of a human. The patient model may not be specific to any patient or is specific to a patient meeting a norm. The patient model includes one or more internal organs and/or one or more tissues. The patient model may be an annotated 3D model with annotated data comprising tissue information, which is used to obtain a radiation specific weighting factor and a tissue specific weighting factor of at least one internal organ and/or at least one type of tissues within the patient model. Additional information may be used to fine-tune the patient model when processing other available data such as patient weight, water-fat-fraction, information about implants etc. Such information may be retrieved e.g. from electronic medical records but can also be measured as part of the patient preparation.

In some examples, besides the patient there may be one or more additional X-ray scattering objects. For example, there may be one or more devices attached to the patient which are inside the field of view of the X-ray but not inside the patient. Examples of such devices may include, but are not limited to, orthosis, tube, cables, and the like. Such additional X-ray scattering objects may also include an X-ray scattering object that supports and/or fixates an anatomy of the patients, e.g., a pillow. In such cases, the distance of the patient and the device (e.g., cables, orthosis, etc.) to the X-ray detector cannot be derived from the 2D projection data, but may have a big impact on the scatter estimation. In order to determine the distance of the device to the X-ray detector, the camera image data of the object may be analyzed to determine the presence of an additional X-ray scattering object. In response to determining the presence of the additional X-ray scattering object, distance information of the additional X-ray scattering object to the detector of the X-ray imaging apparatus is determined. Afterwards, image segmentation may be performed and the distance of the device to the X-ray detector may be determined. Such information may be added to the scatter estimation module to for a better scatter estimation.

In step S330, a scatter profile of scatter present in the X-ray image data is estimated by use of the determined geometry information of the object and distance information of the object to the detector of the X-ray imaging apparatus.

One embodiment to estimate the scatter profile of scatter present in the X-ray image data is the use of Monte-Carlo simulations. Monte-Carlo models that take into consideration the operational parameters, and geometry can effectively determine the characteristics of scattered X-rays. Therefore, given additional information from the camera image data, additional parameters derived from the camera image data can be added to the Monte-Carlo simulations for a more accurate scatter estimation.

Another embodiment to estimate the scatter profile of scatter present in the X-ray image data is the use of a kernel-based scatter estimation approach. An example of the kernel-based scatter estimation method is described in WO2007/148263 A1. The existing approach simulates scatter radiation and parameterizes the results that just uses attention from the X-ray image. In the method as disclosed herein, the camera derived information may be added to the kernel-based scatter estimation module by modifying the height and width of the primitive geometries such as cuboids or ellipsoids delivering scatter estimates, changing the mean density of the material inside the primitive geometries, providing asymmetric shape of the primitive geometries, and/or changing the distance of the primitive geometries to the X-ray detector. The camera derived information may include the determined geometry information of the object and the determined distance information of the object to the detector of the X-ray imaging apparatus. Optionally, the camera derived information may include material probabilities at given position, such as bone, soft tissue, water predicted from a patient model comprising annotated data with tissue information. As a further option, if there is an additional X-ray scattering object, the additional parameters may include the distance of the additional X-ray scattering object to the X-ray detector.

A further embodiment to estimate the scatter profile of scatter present in the X-ray image data is the use of a neural network. The neural network has been trained to reproduce the scatter profile given a function of the X-ray image data and the determined geometry information of the object and the determined distance information of the object to the detector of the X-ray imaging apparatus. The existing algorithm, such as the neural network described in Joscha Maier et al: Real-time scatter estimation for medical CT using the deep scatter estimation: Method and robustness analysis with respect to different anatomies, dose levels, tube voltages, and data truncation. Med Phys. 2019; 46(1); 238-249, uses a neural network that has been trained to produce the output of Monte-Carlo simulations given a function of the X-ray projection data. Given additional information from the camera image, additional parameters, such as determined geometry information of the object and the determined distance information of the object to the detector of the X-ray imaging apparatus, may be provided as a further input to the neural network for a more accurate scatter prediction. The additional parameters may further comprise information like material probabilities at given position, and distance of the additional X-ray scattering object to the X-ray detector.

In an example, the camera derived information may also be used to support image-based scatter correction methods in 3D imaging applications. For example, it is possible to perform a first pass reconstruction and estimating the scatter profile from the first pass reconstruction by using the determined geometry information of the object and the determined distance information of the object to the detector of the X-ray imaging apparatus. Accordingly, the extended field-of-view of the camera can be used to spatially extend the truncated first pass reconstruction used to calculate scatter. The estimated scatter profile is usable for scatter compensation of the X-ray image data before a second pass reconstruction is performed.
The determined scatter profile is output to e.g., a scatter correction device or a storage device.

Optionally, as shown in Fig. 8, the method may further comprise step S340, in which scatter correction is performed based on the scatter profile of scatter present in the X-ray image data. The corrected X-ray image data is then output. The corrected X-ray image data may then be visualized, stored or otherwise (further) processed.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An image processing system (100), comprising:
- an input interface (102) configured to receive X-ray image data of an object acquired by an X-ray imaging apparatus during a scan and to receive camera image data of the object acquired by a camera system having a field of view of the object;
- a geometry determiner (104) configured to analyze the camera image data of the object to determine geometry information of the object and distance information of the object to a detector of the X-ray imaging apparatus; and
- a scatter estimation module (106) configured to estimate a scatter profile of scatter present in the X-ray image data by use of the determined geometry information of the object and the determined distance information of the object to the detector of the X-ray imaging apparatus.

2. The image processing system according to claim 1,
wherein the geometry determiner is configured to fit an object model to the object in the camera image data to determine the geometry information of the object and the distance information of the object to the detector, wherein the object model comprises one or more geometric parameters describing a geometry of the object.

3. The image processing system according to claim 1,
wherein the object model comprises a patient model comprising annotated data with tissue information.

4. The image processing system according to claim 3,
wherein the patient model is adapted according to patient data.

5. The image processing system according to claim 4,
wherein the patient data comprises one or more of the following data:
- patient weight;
- water-fat-fraction; and
- information about an implant.

6. The image processing system according to any one of the preceding claims,
wherein the geometry determiner is configured to analyze the camera image data of the object to determine a presence of an additional X-ray scattering object and in response to determining the presence of the additional X-ray scattering object, to determine distance information of the additional X-ray scattering object to the detector of the X-ray imaging apparatus.

7. The image processing system according to claim 6,
wherein the additional X-ray scattering object comprises one or more of the following:
- a device that is attached to a patient; and
- an additional X-ray scattering object that supports and/or fixates an anatomy of a patient.

8. The image processing system according to any one of claims 1 to 7,
wherein the scatter estimation module is configured to estimate the scatter profile of scatter present in the X-ray image data by use of Monte-Carlo simulations.

9. The image processing system according to any one of claims 1 to 7,
wherein the scatter estimation module is configured to estimate the scatter profile of scatter present in the X-ray image data by use of a kernel-based scatter estimation approach.

10. The image processing system according to any one of claims 1 to 7,
wherein the scatter estimation module is configured to estimate the scatter profile of scatter present in the X-ray image data by use of a neural network, wherein the neural network has been trained to reproduce the scatter profile given a function of the X-ray image data and the determined geometry information of the object and the determined distance information of the object to the detector of the X-ray imaging apparatus.

11. The image processing system according to any one of claims 1 to 7,
wherein the scatter estimation module is configured to estimate the scatter profile of scatter present in the X-ray image data by performing a first pass reconstruction and estimating the scatter profile from the first pass reconstruction by use of the determined geometry information of the object and the determined distance information of the object to the detector of the X-ray imaging apparatus, wherein the estimated scatter profile is usable to for scatter compensation of the X-ray image data before a second pass reconstruction is performed.

12. The imaging processing system of any one of the preceding claims, further comprising:
an exposure determination module configured to estimate a scatter profile from the camera image data prior to the scan, and to determine one or more of a kilovoltage, a milliamperage, and an exposure time based on the scatter profile estimated from the camera image data.

13. The image processing system of any one of the preceding claims, further comprising:
a scatter correction module configured to perform scatter correction based on the scatter profile of scatter present in the X-ray image data.

14. An image processing method, comprising:
receiving (S310) X-ray image data of an object acquired by an X-ray imaging apparatus during a scan and to receive camera image data of the object acquired by a camera system having a field of view of the object;
analyzing (S320) the camera image data of the object to determine geometry information of the object and distance information of the object to a detector of the X-ray imaging apparatus; and
estimating (S330) a scatter profile of scatter present in the X-ray image data by use of the determined geometry information of the object and distance information of the object to the detector of the X-ray imaging apparatus.

15. A computer program element, which, when being executed by at least one processing unit, is adapted to cause the processing unit to perform the method as per claim 14.
